Europäisches Patentamt

European Patent Office

Office européen des brevets

(11) Veröffentlichungsnummer: **0 409 032 A2**

# EUROPÄISCHE PATENTANMELDUNG

(21) Anmeldenummer: 90113059.1

(22) Anmeldetag: 09.07.90

(51) Int. Cl.5: **C12Q 1/00**, C12Q 1/54, C12Q 1/26, C12Q 1/40, G01N 21/77, A61B 5/00

(30) Priorität: 19.07.89 DE 3923921

(43) Veröffentlichungstag der Anmeldung:
23.01.91 Patentblatt 91/04

(84) Benannte Vertragsstaaten:
BE CH DE DK FR GB IT LI NL SE

(71) Anmelder: Gesellschaft für
Biotechnologische Forschung mbH (GBF)
Mascheroder Weg 1
D-3300 Braunschweig-Stöckheim(DE)

(72) Erfinder: Schaffar, Bernhard, Dr.
Mascheroder Weg 1
D-3300 Braunschweig(DE)
Erfinder: Kalisz, Henryk M.
Mascheroder Weg 1
D-3300 Braunschweig(DE)
Erfinder: Sharma, Ashutosh
Mascheroder Weg 1
D-3300 Braunschweig(DE)

(74) Vertreter: Boeters, Hans Dietrich, Dr. et al
Boeters & Bauer Bereiteranger 15 15
D-8000 München 90(DE)

(54) Optischer Biosensor.

(57) Die Erfindung hat einen optischen Biosensor zum Gegenstand. Dieser umfaßt ein Enzym, an das ein Indikator gebunden ist. Während eine von Enzym katalysierte Reaktion im Analyten stattfindet, zeigt der Indikator eine von der Konzentration des zu bestimmenden Analytbestandteils abhängige Indikatorgröße an. Diese kann mittels einer nachgeschalteten Meßanordnung erfaßt werden. Der erfindungsgemäße Biosensor umfaßt vorzugsweise als Enzym Glucoseoxidase, an die ein Fluoreszenzfarbstoff kovalent gebunden ist, der eine von der Sauerstoffkonzentration abhängige Fluoreszenz aufweist, Diese markierte Enzym wird vorzugsweise an einem Trägermaterial immobilisiert.

Fig. 1

EP 0 409 032 A2

## OPTISCHER BIOSENSOR

Die Erfindung bezieht sich auf einen optischen Biosensor.

Der Erfindung liegt die Aufgabe zugrunde, einen Sensor zur Bestimmung eines Analyts vorzusehen, der vielseitig einsetzbar ist und insbesondere für Messungen in Durchflußkammern und direkt in der Lösung und zur Bestimmung von Glucosekonzentrationen geeignet ist.

Diese Aufgabe ist bei einem optischen Biosensor gelöst, der ein Enzym mit einem an dieses gebundenen Indikator aufweist, der die Änderung einer Indikatorgröße während einer vom Enzym katalysierten Reaktion im Analyten wiedergibt. Vorteilhafte Weitergestaltungen des erfindungsgemäßen optischen Biosensors sind Gegenstand der Unteransprüche.

Bei dem erfindungsgemäßen Biosensor dient das Enzym dazu, eine Reaktion im Analyten zu katalysieren. Das Erfassen dieser Reaktion ermöglicht der an das Enzym gebundene, d.h. dieser markierende Indikator, der eine für die Reaktion charakteristische Indikatorgröße anzeigt. Selbstverständlich wird die Auswahl des jeweiligen Indikators so getroffen, daß seine Anzeige eine quantitative Bestimmung eines Analytbestandteils ermöglicht. So wird beispielsweise zur Bestimmung von Glucosekonzentrationen als Enzym Glucoseoxydase verwendet, an die als Indikator ein Fluoreszenzfarbstoff gebunden ist, der eine von der Sauerstoffkonzentration abhängige Fluoreszenz zeigt. Durch das Massen der Fluoreszenzintensität des farbstoffmarkierten Enzyms kann eine Bestimmung der Glucosekonzentration vorgenommen werden, da während der vom Enzym katalysierten Reaktion von diesem Sauerstoff verbraucht wird. So wandelt die zur Glucosebestimmung verwendete Glucoseoxidase Glucose und Sauerstoff in Gluconolacton und Wasserstoffperoxyd um. Mit steigender Glucosekonzentration im Analyten nimmt der Sauerstoffverbrauch während dieser Reaktion zu, womit entsprechend die Fluoreszenzintensität größer wird. Was die Sauerstofflöschung der Fluoreszenz anbelangt, so führt viel Sauerstoff zu einer geringen Fluoreszenz, wenig Sauerstoff hingegen zu einer starken Fluoreszenz. Mit dem erfindungsgemäßen Biosensor ist es möglich, die Konzentration eines Analytbestandteils in einer Lösung direkt zu bestimmen. Zu diesem Zweck braucht das markierte Enzym lediglich in eine Probenflüssigkeit gegeben zu werden. Im Fall von mit Fluoreszenz markierter Glucoseoxidase wird dann die Fluoreszenz vor und nach Zugabe einer Analytlösung, die somit Glucose enthält, gemessen. Wird beispielsweise eine 3mM-Glucoselösung zugegeben, so kann z.B. eine Fluoreszenzzunahme von 60% festgestellt werden.

Vorzugsweise wird das markierte Enzym an einem Trägermaterial immobilisiert, um eine Sensorfläche zu erhalten. Für optische Zwecke wird daher das Enzym gemäß einem vorteilhaften Ausführungsbeispiel der Erfindung auf einem transparenten Träger aufgebracht. Bei dem transparenten Träger kann es sich um eine Glasplatte handeln. Stattdessen können jedoch auch andere Träger verwendet werden, z.B. Kunststoffolien, bzw. wird das markierte Enzym direkt auf das Ende eines Lichtleiters (Einzelfaser oder Faserbündel) aufgebracht. Ein solcher Biosensor kann direkt zur Messung in einer Durchflußmeßkammer eingesetzt werden. Die Montage des Biosensors erfolgt dabei so, daß das markierte immobilisierte Enzym, z.B. Glucoseoxidase, zur Probenlösung weist, während der Träger zu einer zweckmäßig vorgesehenen Fluoreszenzmeßanordnung, einem Fluorimeter, hin gerichtet ist. Die Fluoreszenz des markierten Enzyms wird z.B. mittels Licht mit der Wellenlänge von 405 nm durch den transparenten Träger angeregt und die Fluoreszenz beispielsweise bei 450 nm gemessen. Es können Ansprechzeiten von etwa 50 bis 100 Sekunden erzielt werden. Mit Immobilisierung des Enzyms nimmt die maximale Änderung der Fluoreszenzintensität auf etwa 20% ab (im Vergleich zu 50% bei direkter Messung in Lösung). Dies beruht darauf, daß das von der nachgeschalteten Fluoreszenzmeßanordnung erfaßte Meßsignal einen höheren Störsignalanteil hat. Dieser umfaßt Streulicht und Untergrundfluoreszenz, so daß die prozentuale Änderung des Gesamtsignals mit Änderung der Konzentration der zu erfassenden Meßgröße kleiner ist. Es können im Fall der Glucosekonzentration Bestimmungen im Bereich von etwa 0,01 bis 2 mM durchgeführt werden. Die Sensoransprechzeit ist hierbei jedoch von den jeweils verwendeten Immobilisierungsverfahren abhängig.

Vorzugsweise wird als Fluoreszenzindikator Dekacyclen verwendet. Es kann jedoch jeder beliebige Fluoreszenzfarbstoff verwendet werden, der eine Änderung der Fluoreszenzintensität mit der Sauerstoffkonzentration zeigt.

Die Enzymschicht ist vorteilhaft mit einem lichtabsorbierenden Stoff, vorzugsweise einer Schicht auf Aktivkohle, überzogen.

Erfindungsgemäß kann die Enzymschicht vorteilhaft mit einer Schutzmembran versehen sein, die zweckmäßig durch Tauchen in eine Lösung aufgebracht ist. Diese verhinder unerwünschte Reaktionen des Enzyms mit der Probenflüssigkeit. Entsprechend der Dicke der Schutzmembran kann die Sensorempfindlichkeit bzw. der analytische Bereich des Sensors vorteilhaft eingestellt werden. Es wird vorzugsweise eine Schutzmembran aus Celluloseacetat (Celluloseacetatlösung in Acetonitril) oder Cuprophan verwendet. Es können jedoch auch andere Schutzmembranmaterialien engesetzt werden. Der lichtabsorbierende Stoff wird vorzugsweise zusammen mit der Schutzmembran aufgebracht.

Wie erwähnt ist das Enzym bei einer bevorzugten Ausführungsform der Erfindung Glucoseoxidase. Alternativ kann das Enzym beispielsweise eine der Oxidasen, Oxygenasen oder Oxidoreductasen sein. Dies ermöglicht die Bestimmung weiterer Analyste zusätzlich zur Glucosebestimmung.

Als Fluoreszenzfarbstoff ist vorzugsweise 3-COOH,-7-OH-Cumarin vorgesehen, das nach dem Carbodiimid-Verfahren kovalent an das Enzym (z.B. Glucoseoxidase) gebunden wird. Weiterhin bevorzugt wird 3-COOH-Succinimidylester-7-OH-Cumarin verwendet, das direkt kovalent mit dem Enzym verbunden werden kann. Hierbei werden jeweils zwischen den Carboxygruppen des Farbstoffes und den Aminogruppen des Enzyms Peptidbindungen ausgebildet.

Als Immobilisierungstechnik wird vorzugsweise die Immobilisierung mit Polyvinylalkohol verwendet, wobei das markierte Enzym, z.B. mit 7-Hydroxycumarin markierte Glucoseoxidase, in einem Hydrogel aus photovernetztem Polyvinylalkohol immobilisiert wird. Es können jedoch auch andere Immobilisierungstechniken angewendet werden, beispielsweise ist eine Quervernetzung mit Glutardialdehyd möglich. So kann jede Immobilisationstechnik eingesetzt werden, bei der das Enzym in einer Art Hydrogel unter Erhalt der Enzymaktivität gebunden wird. Beispielsweise ist das Einbringen eines markierten Enzyms in ein Polyacrylamidgel möglich.

Ein erfindungsgemäß vorgesehenes bevorzugtes Verfahren zur Herstellung eines optischen Biosensors umfaßt die Schritte, daß ein Lösung eines Enzyms mit einem an dieses gebundenen Indikator hergestellt wird, die Lösung mit Polyvinylalkohol-Lösung gemischt wird, die Lösungsmischung auf einen transparenten Träger aufgebracht wird und die Lösungsmischung getrocknet und photovernetzt wird. Ein so hergestellter Biosensor kann direkt in einer Durchflußmeßkammer verwendet werden.

Es können auch andere Indikatorgrößen mit dem erfindungsgemäßen Biosensor erfaßt werden. Beispielsweise kann statt Messung des Sauerstoffverbrauchs z.B. während einer Reaktion z.B. die Änderung des pH-Wertes festgestellt werden. Hierzu müssen pH-Indikatoren direkt am Enzym immobilisiert werden. Im Fall einer pH-Bestimmung können auch andere Meßanordnungen als Fluoreszenzmeßanordnungen verwendet werden, z.B. eine Reflexions- oder Absorptionsmeßanordnung.

Die Erfindung wird im folgenden weiter anhand bevorzugter Ausführungsbeispiele und der Zeichnung beschrieben. In der Zeichnung zeigen:

Fig. 1 ein Diagramm, das die Änderung der relativen Fluoreszenz durch Zugabe von Glucose zu einer Lösung des markierten Enzyms,

Fig. 2 ein Diagramm, das eine Kalibrationskurve der relativen Fluoreszenz in bezug auf die Glucosekonzentration für einen Biosensor mit immobilisiertem Enzym veranschaulicht und

Fig. 3 den Aufbau eines Glucose-Biosensors mit immobilisiertem Enzym.

Die Erfindung wird im folgenden weiter anhand von Beispielen mit Glucose-Biosensoren erläutert. Selbstverständlich können wie erwähnt andere Analytbestandteile mit dem erfindungsgemäßem Biosensor bestimmt werden. Bei den Ausführungsbeispielen ist stets als Enzym mit 7-Hydroxycumarin markierte Glucoseoxidase verwendet und es wird die Fluoreszenzintensität gemessen.

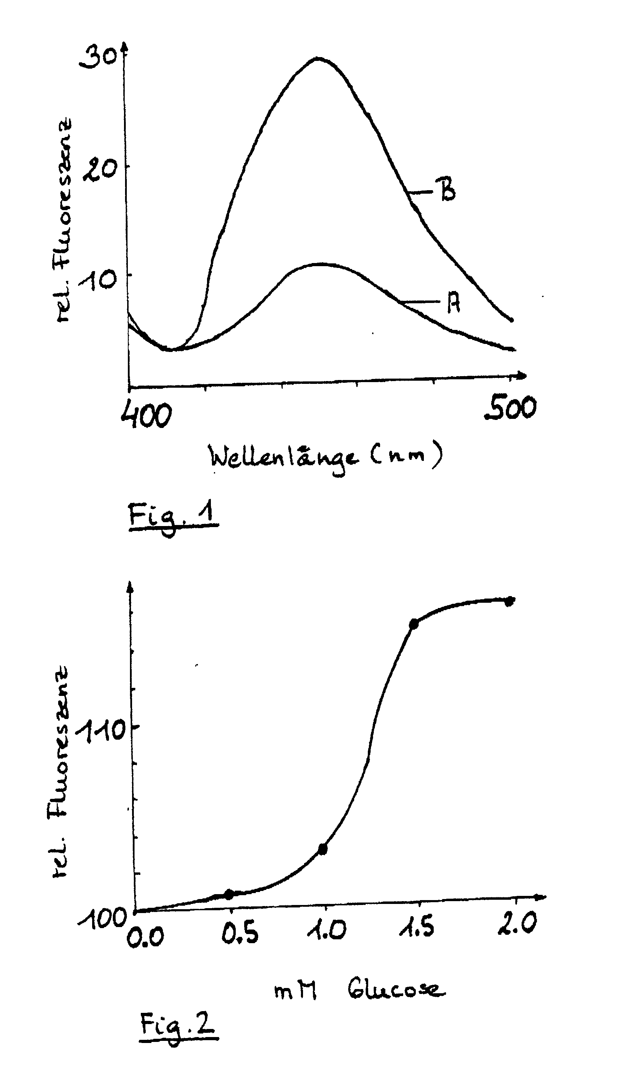

Beim Beispiel von Fig. 1 wird ein Biosensor ohne Träger verwendet, d.h. das markierte Enzym wird in der Probenlösung gelöst. Im Diagramm sind zwei Meßkurven A bzw. B dargestellt, die die relative Fluoreszenz in Abhängigkeit von der Wellenlänge veranschaulichen. Die Meßkurve A stellt das Fluoreszenspektrum ohne Zugabe von Glucose dar. Die Meßkurve B ist nach Zugabe von 3 mM Glucoselösung aufgenommen worden. Ein Vergleich der Kurven A und B zeigt, daß die relative Fluoreszenz mit Glucosezugabe um 60% angestiegen ist. Diese Art der Glucosebestimmung kann als kinetisches Verfahren bezeichnet werden.

Die in Fig. 2 dargestellte Kalibrationskurve zeigt die relative Fluoreszenz in Abhängigkeit von der Glucosekonzentration für einen Biosensor mit auf einem Träger immobilisiertem markiertem Enzym. Bei dem veranschaulichten Ausführungsbeispiel ist das markierte Enzym in einem Hydrogel aus photovernetztem Polyvinylalkohol immobilisiert worden. Wie veranschaulicht ist, ändert sich die Fluoreszenzintensität um 20%. Diese Änderung ist deutlich geringer als bei dem in Fig. 1 veranschaulichten Ausführungsbeispiel. Dies beruht darauf, daß das optische Meßsignal zusätzlich Streulicht und Untergrundfluoreszenz enthält, die die prozentuale Änderung des Gesamtsignals herabsetzen. Die in Fig. 2 veranschaulichte Kalibrationskurve ist nichtlinear. Eine Bestimmung der Glucosekonzentration im Bereich von etwa 0,01 bis 2 mM ist jedoch gut möglich.

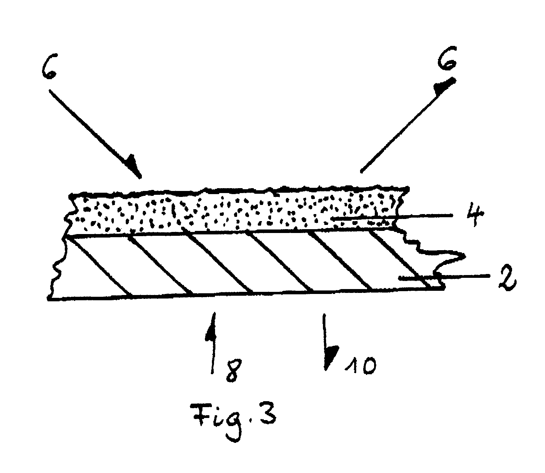

In Fig. 3 ist ein Beispiel für den Aufbau eines Glucose-Biosensors veranschaulicht. Der Sensor weist einen Träger 2 auf, bei dem es sich im veranschaulichten Ausführungsbeispiel um ein Glasplättchen handelt. Auf das Glasplättchen ist ein markiertes Enzym im Bereich von 1 cm$^2$ aufgetragen. Zu diesem Zweck ist einer Lösung von 400 U/ml markierter Glucoseoxidase ein Aliquot von 50 U/ml entnommen worden und im Verhältnis von 1:2 mit einer fertigen Polyvinylalkohol-Lösung gemischt worden. Nach dem Auftragen ist die Anordnung getrocknet und photovernetzt worden. Der so hergestellte Biosensor kann direkt in einer Durchflußmeßkammer eingespannt werden. Die Pfeile 6 veranschaulichen die Probenflüssigkeit, mit der das Enzym katalytisch reagiert. Mittels der Pfeile 8, 10 ist das durch den Träger 2 zugeführte Licht zur Anregung der Fluoreszenz und das zurückkommende Meßsignal veranschaulicht. Zur Anregung wird Licht mit der Wellenlänge von 405 nm verwendet. Die Messung der Fluoreszenz erfolgt bei 450 nm.

**Ansprüche**

1. Optischer Biosensor, **gekennzeichnet** durch ein Enzym (4) mit einem und dieses gebundenen Indikator, der eine Indikatorgröße während einer vom Enzym katalysierten Reaktion im Analyten (6) anzeigt.

2. Optischer Biosensor nach Anspruch 1, dadurch **gekennzeichnet**, daß das Enzym (4) auf einem transparenten Träger (2) aufgebracht ist.

3. Optischer Biosensor nach Anspruch 2, dadurch **gekennzeichnet**, daß der Träger (2) eine Glasplatte ist.

4. Optischer Biosensor nach Anspruch 2, dadurch **gekennzeichnet**, daß der Träger eine Kunststoffolie ist.

5. Optischer Biosensor nach einem der Ansprüche 1 bis 4, zur Bestimmung von Glucosekonzentrationen, dadurch **gekennzeichnet**, daß das Enzym (4) Glucoseoxidase ist.

6. Optischer Biosensor nach einem der Ansprüche 1 bis 5, dadurch **gekennzeichnet**, daß das Enzym aus den Oxidasen, Oxygenasen und Oxidoreductasen gewählt ist.

7. Optischer Biosensor nach einem der Ansprüche 1 bis 6, dadurch **gekennzeichnet**, daß der Indikator ein Fluoreszenzfarbstoff ist, der eine von der Sauerstoffkonzentration abhängige Fluoreszenz zeigt.

8. Optischer Biosensor nach Anspruch 7, dadurch **gekennzeichnet**, daß der Fluoreszenzfarbstoff 3-COOH,-7-OH-Cumarin oder 3-COOH-Succinimidylester-7-OH-Cumarin ist.

9. Optischer Biosensor nach einem der Ansprüche 1 bis 6, dadurch **gekennzeichnet**, daß der Indikator ein pH-Indikator ist.

10. Optischer Biosensor nach einem der Ansprüche 1 bis 9, dadurch **gekennzeichnet**, daß das Enzym (4) durch Einbringen eines Hydrogels aus photovernetztem Polyvinylalkohol immobilisiert ist.

11. Optischer Biosensor nach einem der Ansprüche 1 bis 10, dadurch **gekennzeichnet**, daß die Enzymschicht mit einem lichtabsorbierenden Stoff überzogen ist.

12. Optischer Biosensor nach einem der Ansprüche 1 bis 11, dadurch **gekennzeichnet**, daß auf der Enzymschicht eine Schutzmembran angeordnet ist.

13. Optischer Biosensor nach Anspruch 12, dadurch **gekennzeichnet**, daß die Schutzmembran aus Celluloseacetat oder Cuprophan ist.

14. Optischer Biosensor nach Anspruch 12 oder 13, dadurch **gekennzeichnet**, daß die Sensorempfindlichkeit mittels der Dicke der Schutzmembran einstellbar ist.

15. Optischer Biosensor nach einem der Ansprüche 1 bis 14, dadurch **gekennzeichnet**, daß eine Fluoreszenzmeßanordnung vorgesehen ist.

16. Optischer Biosensor nach Anspruch 9, dadurch **gekennzeichnet**, daß eine Reflexions- oder Absorptionsmeßanordnung vorgesehen ist.

17. Verfahren zur Herstellung eines optischen Biosensors, insbesondere nach einem der Ansprüche 2 bis 16, dadurch **gekennzeichnet**, daß
- eine Lösung eines Enzyms mit einem und dieses gebundenen Indikator hergestellt wird,
- diese Lösung mit einer Polyvinylalkohol-Lösung gemischt wird,
- die Lösungsmischung auf einem transparenten Träger aufgebracht wird und
- die Lösungsmischung getrocknet und photovernetzt wird.

Fig. 1

Fig. 2

Fig. 3